# EUROPEAN PATENT APPLICATION

(11) **EP 4 296 352 A1**
(43) Date of publication of application: **27.12.2023**
(21) Application number: 22179991.9
(22) Date of filing: 20.06.2022
(51) Int. Cl.: C12M 3/08, C12M 1/42, C12M 1/33, C12M 1/00

(54) **TUNABLE DISRUPTION OF EUKARYOTIC PROTOPLAST TO RELEASE INTACT CELLULAR ORGANELLES**

(71) Applicant: LenioBio GmbH, 40231 Düsseldorf (DE)
(72) Inventor: Jürgens, Hannes, Aachen (DE); Lamm, Robin, Aachen (DE)
(74) Representative: Heide, Anna Katharina

(57) **Abstract**

The present invention relates to an apparatus and method using said apparatus for disruption of cell wall free cells without disrupting comprising biologically active compartments enclosed by a lipid bilayer, wherein a shear force generating device (S) generates a region of a shear force suitable for disrupting the cells. Optionally, a pump (P) for pumping an aqueous medium comprising said cells with an adjustable flow rate or flow velocity, preferably adjusting the residence time and the shear force separately, and a separation device (C) is combined for separating the at least one released biologically active compartment and/or its surrounding biologically active liquid phase from debris. The apparatus is a standalone system or an integral part of a production line. Said apparatus and method enable the production of products comprising a high yield of biologically active compartments exhibiting a high biological activity, in particular in energy regeneration and/or protein synthesis.

## Description

The present invention relates to an apparatus and a method using said apparatus for disruption of cell wall free cells without disrupting comprising biologically active compartments enclosed by a lipid bilayer, wherein a shear force generating device (S) generates a region of a shear force suitable for disrupting the cells. Optionally, a pump (P) for pumping an aqueous medium comprising said cells with an adjustable flow rate and/or flow velocity and a separation device (C) for separating the disrupted cells and fragments thereof from the at least one released biologically active compartment and/or its surrounding biologically active liquid phase is combined. The apparatus is a standalone system or an integral part of a production line. Said apparatus and methods enable the production of a composition comprising a high yield of biologically active compartments exhibiting a high biological activity, in particular in energy regeneration and/or protein synthesis.

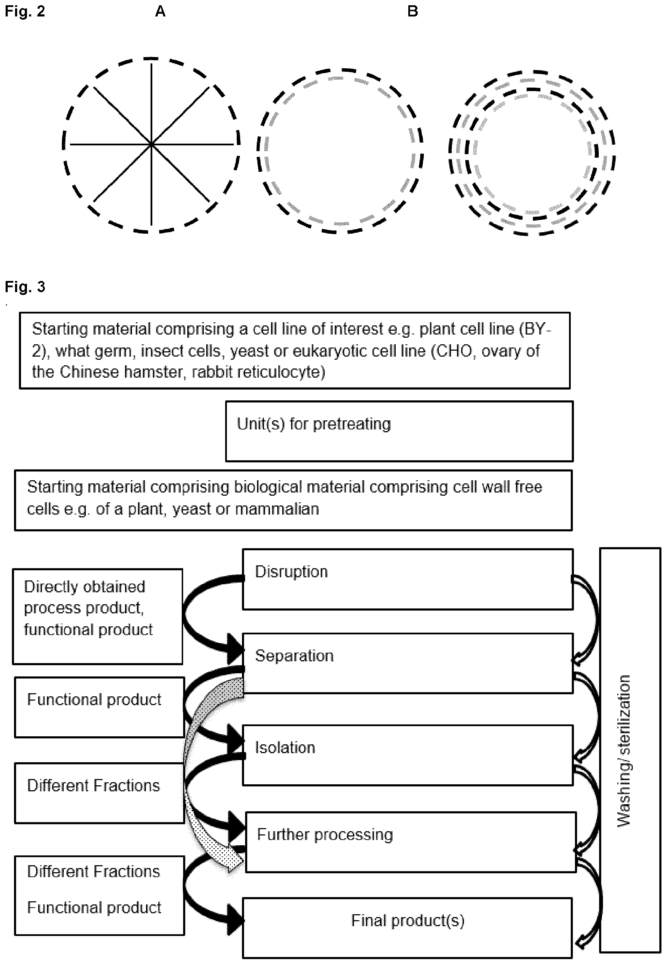

In the technical field of cell culture, in particular for the production of cell lysates from living cells for cell free production process, e.g. of recombinant proteins, different methods are used to generate the lysate from the cells. In particular for protein biosynthesis based in native systems, it is desired to use cell free systems for a number of reasons in order to avoid: later difficult extraction of proteins from the living cells, (lengthy) individual cell line development for each desired target protein, long cultivation times, low protein production rates. Therefore, it is desired to extract the desired machinery from the cells, such as the protein machinery, and to use it for recombinant protein biosynthesis. For the cell free protein synthesis, extraction of microsomal vesicles derived from the endoplasmic reticulum and Golgi, promoting the formation of disulphide bonds, glycosylation and the co-translational integration of membrane proteins, is required. Further, it is also desired to extract mitochondria providing the energy for the biosynthesis of proteins. In order to achieve high yield of proteins and a fast biosynthesis, a high concentration of the aforementioned biologically active compartments is advantageous. In the prior art, in particular the method of homogenization, e.g. Dounce homogenizer (Braun, Melsungen, Germany), is used to break up the cell wall free cells. Thereafter non-disrupted cells, nuclei and cell membrane fragments are removed to achieve the desired lysate for protein synthesis. However, this device is not suitable for an inline-production, not scalable and thus achieves low yields of the lysate. Further, valve homogenization results in particles in the range of 0,5-1 µm (Zhang et al., table 1, Fig. 3) which is expected to damage the desired microsomes and the not desired nucleus. In particular, the presence of plant DNA affects the biosynthesis of the recombinant protein as the plant DNA will also be targeted and native plant proteins will be produced instead of the recombinant protein target. WO9712959A1 discloses a method for the disruption of cultured cells which lack a cell wall comprising passing cells suspended in a culture fluid through a low-pressure impinging jet device with the aim to isolate naturally occurring products such as proteins, polysaccharides, recombinant proteins and viruses. However, the method and the device do not enable the separation of biologically active compartments and are significantly different from the present solution.

Therefore, one object of the present invention is to provide a method and an apparatus for a tuneable disruption of eukaryotic cells, in particular of eukaryotic (mini)protoplast to release intact cellular organelles, such as mitochondria and/or microsomes. It is an object to provide an apparatus and a method that allows to disrupt (mini)protoplasts without destruction of cell organelles in an efficient, scalable and inline manner. Another object is to provide an apparatus and a method which allows to adjust and tune the shear force and the residence time in relevant order of magnitude independent from each other. Further another object of the invention is to provide an apparatus that is sterilizable and a method wherein a sterile, closed processing is possible.

Therefore, the present invention provides a solution described below in detail, with the advantages that it enables an adjustable/tuneable shear force and residence time in relevant order of magnitude, via rotor speed and/or flow rate and/or flow velocity, independent from each other. The apparatus and method according to the present invention further enables a closed and sterile system which is scalable. Thus, so far there was no device and method available that allows scaling of cell disruption that ensures the release of biologically active compartments. Further, none of the so far known devices for particle treatment, sonication, homogenizer or grinder, or other in-line cell disruption devices offer tuneable shear forces in the right order of magnitude. Continuous shear force generating devices, e.g. rotor-stator mixer, are usually to contribute to pumping the fluid and used without external feed pumps. The apparatus according to the present invention, for the first time offer independent control of shear and residence time and enable a reasonable balance between pumping speed and shear force. Thereby, a gentle disruption of cells, in particular of cell cultures, that provides intact organelles, such as biologically active compartments is provided. The advantage of the present invention is, that the lysate obtained by the described method performed by the described apparatus is improved and enables a faster protein production at higher protein yields.

The first aspect of the present invention in an apparatus for disruption of cell wall free cells, preferably of (mini)protoplasts and/or spheroplasts, without disrupting comprising biologically active compartments enclosed by a lipid bilayer, the apparatus comprises
- a shear force generating device (S) for generating a region of a shear force suitable for disrupting the cells,
- a tank A for the provision of the cells, preferably of plant cells, more preferably of a plant of the genus Nicotiana of the family Solanaceae, most preferably the plant is N. tabacum, most preferably a BY-2 cell line from N. tabacum,
- optionally a pump (P) positioned upstream of (S) or downstream of (S) for pumping an aqueous medium comprising said cells with an adjustable flow rate and/or flow velocity,
- at least one conduit connecting the tank A and the shear force generating device S and/or at least one conduit connecting (S) with a tank B,
- a tank B for collecting a product from the previous steps comprising at least one released biologically active compartment, and
- a separation device (C) to separate the disrupted cells and fragments thereof from the at least one released biologically active compartment and/or its surrounding biologically active liquid phase.

In an embodiment of the apparatus according to the present invention the shear force generating device (S) is suitable and controllable to generate shear forces sufficient to disrupt the cells without damaging the lipid bilayer of the at least one comprising biologically active compartment.

Preferably, the applied shear force does not damage the "biological active content". Preferably (S) is a centrifugal pump, a high shear forces mixer or a Rotor-Stator-mixer with a teeth-design or with a blade-design (Fig. 2). The rotor-stator system containing a rotor with a teeth-design consists of a round plate mounted on rotating shaft with one or more circles of teeth. Together with the stator, one or more sets of concentric teeth circles make up the rotor-stator system (Fig. 2 B). However, different designs of the shear force generating component of the shear force generating device (S) are possible.

In one embodiment, the shear force generating device (S) is a rotor-stator system which are suitable in-line production embodiments of the present invention (Håkansson, 2018). Suitable rotor-stator systems are commercially available, e. g. IKA magic LAB and the IKA UTL 25 inline, depending on the design the rotor-stator system is adjustable within a speed range [min-1] of 3.000 - 26.000, 10000-24000, 3000-20.000, adjustable to a throughput of 2-20 l/min, 5-15 l/min, 5-12 l/min, 11.6 l/min up to [l/h] of 1 - 1500 [l/h], 1-100 [l/h], 10-1000 [l/h], 10-200 [l/h], 30 - 200 [l/h], 50 - 200 [l/h], 1 - 20 [l/h], up to 1500,1-20 [kg/h] and/or adjustable to a circumferential speed [m/s] of 5 - 40, 5-30, 5-23 and 6-16. Within the meaning of the invention, the speed of the rotor-stator system, preferably for an aqueous medium, is at least 3000 rpm, at least 4000 rpm, at least 4500 rpm, 5000 rpm, at least 6000 rpm, at least 7000, at least 8000 rpm, at least 9000, at least 10000 rpm or more or any other speed between the aforementioned ranges. The speed is dependent on the scaling of the disruption method according to the present invention and dependent on the used apparatus, stand-alone system or preferably the in-line embodiment of the apparatus according to the present invention. Therefore, the present invention encompasses any adjusted speed, optionally or preferably in combination with any adjusted speed of the pump, in order to obtain the desired directly obtained "process product" as defined herein and performed by the apparatus enabling the scaled disruption method.

The "directly obtained process product" by the method according to the present invention, preferably performed by use of the apparatus according to the present invention, is the aqueous medium downstream of the shear force generating device after the at least one disruption circle according to the present invention and comprises disrupted cell wall free cells, preferably disrupted miniprotoplast, protoplast and/or spheroplasts. This directly obtained process product preferably comprises at least one biologically active compartment and its surrounding biologically active liquid. The liquid and/or the at least one compartment enables at least ATP synthesis and/or energy regeneration and/or at least one protein biosynthesis associated process, expression, transcription, translation, translocation, protein folding and/or protein modification. The "biological active content" comprises biological active molecules such as enzymes, soluble proteins and/or small molecules derived from the cytosol of the cell wall free cells (=surrounding biologically active liquid), and/or mitochondria, microsomes, Nucleus, Golgi and/or ER. Thus, the "functional product" is defined by its capability to perform or enable cell free protein biosynthesis of a recombinant protein. Preferably, the functional product comprises at least one released biologically active compartment (part of "biological active content") that is capable of ATP synthesis, energy regeneration, of at least one protein biosynthesis associated process, expression, transcription, translation, translocation, protein folding and/or protein modification. More preferably the "functional product" is separated from debris (nuclei, cell membrane debris, and (mini)protoplast debris) and exhibits the capacity of cell free protein biosynthesis of a recombinant protein. Preferably, the production capacity is more than 10 ml/day up to 100 L/day, up to 1000 L/day. Preferably the apparatus, alone or as integral component of the production is operating automatically.

In another embodiment, the shear generating device is a centrifugal pump, e.g. a Levitronix^{®} puraLev i100 SU (e.g. PLD-i100SU.1, PLD-i100SU.5, PLD-i100SU.2, PLD-i100SU.3). This pump is based on the principles of magnetic levitation, the pump's impeller is suspended, contact-free, inside a sealed casing and is driven by a magnetic field of the motor. The pump-head is a disposal, sterile or sterilizable prior use and suitable for biological material. The flow rate is adjusted by the rotor speed and allows also control of pressure. The max. flow rate is 17 L/min, max. Diff.-Pressure 2 bar / 29 psi and Max. Viscosity of the medium is < 20 cP. The flow rate of a centrifugal pump, preferably for an aqueous medium, is preferably adjustable in the range of 0,00 - 50 mL/min, 0,0 up to 40 L/min, 0,0 up to 30 L/min, 0,0 up to 20 L/min, at least 2,5 L/min up to 50 L/min to 20 L/min, at least 5,0 L/min up to 50 L/min to 20 L/min, at least 7,5 L/min up to 50 L/min to 20 L/min, at least 10 L/min up to 50 L/min to 20 L/min, at least 12,5 L/min up to 50 L/min to 20 L/min, at least 15 L/min up to 20 L/min, at least 17,5 L/min up to 20 L/min. This ranges of flow rate are achievable by a rotor speed in the range of 3000 rpm up to 10000 rpm, 3000 rpm up to 9000 rpm, 4000 rpm up to 9000 rpm, 4000 rpm up to 8000 rpm, 4000 rpm up to 7000 rpm, 4000 rpm up to 6000 rpm and 4000 rpm up to 5000 rpm.

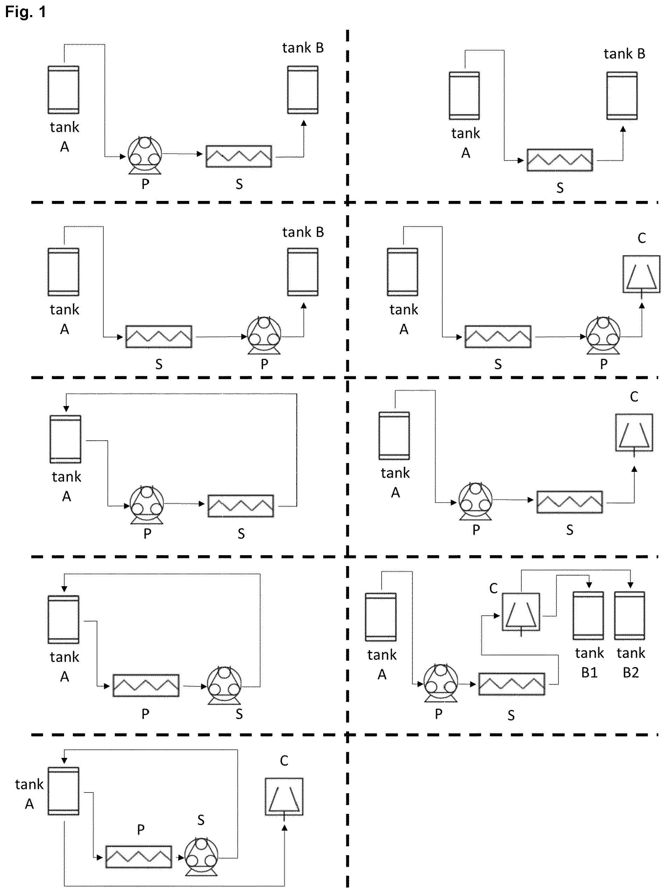

Håkansson, 2018, gives guidance for scaling of rotor-stator system as well as for calculation of the forces in batch and in-line (pages 8-10) and compares the rotor-stator system with centrifugal pumps. Different embodiments of high shear forces mixer (HSM) are disclosed in Zhang et al. which are suitable as a shear generating device according to the present invention. In particular Fig. 1 of Zhang et al. represents different geometric variations of commercial high shear mixers, e.g. the teethed inline unit (Ytron-Quadro z), the blade-screen in-line unit (Silverson 150/250 MS), the radial-discharged units (Left - Silverson L4R, Right - VMI Rayneri) and the axial-discharged unit (Greerco 1.5 HR). These HSM can be used for in-line productions and as stand-alone systems (batch units) as described in Espinoza et al..

Preferably, the production line has a production capacity of at least 10 ml/day, wherein "day" is a production day defined as the period from the end of cell cultivation until obtaining the "functional product".

In another embodiment of the apparatus according to the invention the pump is suitable to adjust a residence time of the aqueous medium comprising the cells within (S) independently from the shear force adjusted and generated by (S), wherein the residence time is the duration for which the shear force is exerted onto the aqueous medium comprising the cells. The "residence time" can be controlled or modulated by the adjustment of flow rate and/or flow velocity of the pump. In combination with the desired geometry of the shear force generating device, preferably of the rotor-stator-mixer, the residence time can be controlled or modulated by the adjustment of flow rate and/or flow velocity of the pump independently from adjustment of the shear force of (S). Thus, the flow rate and/or flow velocity is used to manipulate the residence time.

Flow rate specifies the rate of fluid transport through a system, it is the amount of fluid moved per unit time (e.g. mL/min). Flow velocity specifies the speed with which a fluid moves through a system, measured in distance per time (e.g. m/s). Both flow rate and flow velocity are relevant for the rotor stator system. Flow rate, in combination with geometry of the rotor stator shear force region (specifically the volume of the shear force region), determines residence time in the shear force region. Similarly, flow velocity, in combination with geometry of the rotor stator shear force region (specifically path length through the shear force region), determines residence time in the shear force region.

Preferably, the residence time is the duration for which the shear force is exerted onto the aqueous medium comprising the cells that is sufficient to disrupt the cells without disrupting the at least one comprising biologically active compartments enclosed by a lipid bilayer. More preferably the residence time is the duration for which the cells are retained in the region of shear forces and/or turbulence. The residence is as short as possible in order to accelerate the disruption and the whole production process. Preferably the residence time is less than 25 sec, less than 20, less than 10 sec. In the present example and for the specific apparatus design and process, the residence is less than 10 sec, less than 9, 8, 7, 6, 5, 4, 2, 1 sec. It is preferred to reduce the residence as much as possible which is achievable by adaptation of the design of (S) in respect of geometry and volume in combination with the design of the pump, respectively. Thus, it is clear to the skilled person that for upscaling the apparatus and components thereof have to be adjusted in order to maintain the same residence time as shown in the present examples.

The pump is a preferably a self-priming and/or self-sealing against backflow, positive displacement (e.g. peristaltic) pump with adjustable pump speed. Preferably the pump controls the flow rate and/or flow velocity at which the isotonic or hypertonic aqueous medium is transported into the S, in particular into the space between the rotor and stator. One embodiment comprises a peristaltic pump e.g. Watson marlow 120u/dv with adjustable pump speed in the range between 0 and 200 rpm max was used. However the pump speed (rpm) for this pump is at least 10 rpm, at least 15 rpm, at least 20 rpm, at least 50 rpm, at least 100 rpm, 150 rpm, at least 200 rpm, at least 250 rpm ,at least 300 rpm, at least 500 rpm or any integer between these values. Peristaltic pumps do not contain valves, seals or glands to clog or corrode and therefore are the simplest pump. The aqueous medium contacts only the inner wall and inner space of the conduit. This ensures hygienic processing and no risk of the pump to contaminate the aqueous medium, or that aqueous medium contaminates the pump. The peristaltic pump can be operated at a temperature of at least -10°C up to 50°C and allows operation under controlled temperatures. This pump is suitable for viscous, shear-sensitive aqueous media even containing solid components. According to the present invention the aqueous medium comprises biological material which comprise cells and cell wall free cells which are not considered solid. According to the manufacturer information the pump-head can be adjusted to accommodate 1.6 mm wall tubing in sizes from 0.5 mm inside diameter to 4.8 mm inside diameter. It is clear to the skilled person, that flow rates may vary because of changes in the aqueous medium, viscosity of the aqueous medium, processing temperature, inlet and discharge pressures, system configuration and/or tubing performance against time. In general the flow rate is between 0.001 to 170 ml/min. Some examples of flow rates for different tubing set ups are shown below:

| Speed **[rpm]** | 0.5mm | 0.8mm | 1.6mm | 2.4mm | 3.2mm | 4.0mm | 4.8mm |
|---|---|---|---|---|---|---|---|
| | ml/min | | | | | | |
| 0,1-200 | 0,002-4 | 0,004-8 | 0,01-28 | 0,03-58 | 0,05-97 | 0,07-130 | 0,09-170 |
| 1 | 0,02 | 0,04 | 0,14 | 0,29 | 0,47 | 0,67 | 0,85 |

However, it is recommended to determine flow rates under operating conditions before implementing into the method and apparatus according to the present invention. The pump speed may be adjusted accordingly, if another pump is combined. In particular in case of scaling of the disruption method according to the present invention and dependent on the accordingly used apparatus, stand-alone system or preferably the in-line embodiment of the apparatus, an adjustment of pump size, processing parameter and flow rates will be necessary. Therefore, the present invention encompasses any adjusted pump speed, preferably in combination with any adjusted speed of the shear force generating device, in order to obtain the desired directly obtained "process product" as defined herein and performed by the apparatus enabling the scaled disruption method.

It is difficult to predict the turbulences and shear in a shear force generating device of different scale. In a liquid-liquid approach - it is proposed to apply said approach because cells in a culture and due to the high water content of the cells those can be considered "liquid" - highly localized intense turbulence and shear in shear force generating devices, in particular rotor-stator and HSMs, cause multiple breakage mechanisms which result the disruption of a droplet (Zhang et al with reference to Kolmogoroff cited therein). Said approach can be transferred to a cell wall free cell according to the invention.

In another embodiment of the apparatus according to invention, the apparatus is an integral apparatus of a production line, preferably of a closed and sterile production line, more preferably of a production line for a plant cell line, in particular of a plant cell free lysate production line. In another embodiment of the apparatus according to invention, the apparatus is a stand-alone system, preferably a closed and sterile stand-alone system, more preferably it is a system for disruption of a plant cells. Preferably the plant cell is a plant cell line of N. tabacum, most preferably a BY-2 cell line from N. tabacum.

Thus, the apparatus may be a stand-alone system as schematically shown in Fig. 1. In the stand-alone system tank A provides the starting material. The starting material is an aqueous medium comprising the cell wall free cells, more preferably a cell culture of an animal cell, plant or fungal (mini)protoplasts and bacterial spheroplasts and amenable to a large-scale culture. A cell culture, preferably after a pretreating, more preferably after evacuolation of a plant cell line, to be disrupted as defined herein. The tank A is connected via a conduit with the shear force generating device (S) and a downstream conduit connects the shear generating device (S) with tank B. A pump (P) optionally is integrated into the stand-alone system and may be placed upstream or downstream of the shear generating device (S). Within the stand-alone system a separation device (C), preferably a centrifuge is place downstream of the shear generating device (S), in particular downstream of the pump if one is integrated. Said stand-alone system enables circular run of the disruption. "Circular run" means that the predetermined conditions of the speed of the shear generating device (S) and optionally in combination with the speed of the pump are repeated. The forces are exerted more than once onto the aqueous medium comprising the biological material more than time. According to the invention the aqueous medium passes the region of shear force more than once, if necessary at least twice, three time, four times or more. The disruption according to the present invention may comprise at least one, at least two, at least three, at least four or more passes of at least the step of providing an aqueous medium comprising the cell wall free cells, preferably in a tank A, moving of the aqueous medium into the shear force generating device (S), exerting a shear force onto the aqueous medium comprising said cells and for a residence time that is sufficient to disrupt the cells without disrupting the at least one biologically active compartment and disrupting the cells without disrupting the lipid bilayer of the at least one biologically active compartment while the aqueous medium is passing S.

In the embodiment of the apparatus according to invention, wherein said the apparatus is an integral apparatus of a production line (Fig. 1 applies accordingly, wherein tank A, tank B and/or (C) are coupled to downstream or upstream units), upstreaming tanks, components and/or device for upstreaming seps are possible. Any type of pretreating known to the skilled person in the field of cellular bioreactors, biotechnological bioreactors and production system of cell lines of any species are conceivable, e.g. cell lines of microorganism, human cell lines, yeast, fungal and plant. For each of the production lines it is preferred that it is a closed and sterile production line, more preferably it is automatically operated and controlled. More preferably it is a production line for a plant cell line, a plant cell line of N. tabacum, most preferably a BY-2 cell line from N. tabacum.

Preferably, the production line has a production capacity of at least 10 ml/day, wherein "day" is a production day defined as the period from the end of cell cultivation until obtaining the "functional product". The "functional product" is defined by its capacity to perform or enable cell free protein biosynthesis of a recombinant protein. Preferably the functional product comprises at least one released biologically active compartment that is capable of ATP synthesis, energy regeneration, and/or of at least one protein biosynthesis associated process, expression, transcription, translation, translocation, protein folding and/or protein modification. More preferably the "functional product" is separated from debris (cell membrane debris, and (mini)protoplast debris) and exhibits the capacity of cell free protein biosynthesis of a recombinant protein. Preferably, the production capacity is more than 10 ml/day up to 100 L/day, up to 1000 L/day. Preferably the apparatus, alone or as integral component of the production is operating automatically. The stand-alone system has a capacity of at least 100 ml, at least 1 L in tank (A), at least 1L, at least 5L, at least 10L, at least 100L, at least 1000L and is adjustable to any desired process volume. In particular the scaling is adjustable dependent on the predetermined volume of tank (A), the volume the shear generating device (S) can process at the same time and the volume the optional pump (P) can process at the same time.

Another aspect of the present invention is a production line comprising the apparatus according to the present invention, wherein tank (A) is placed downstream of at least one unit for a pretreating of a biological material and the conduit connects tank (A) with (S) and wherein tank (B) is placed downstream of (S) and optionally a pump (P) is placed downstream or upstream of (S). As described therein "pretreating" comprises any other treatment of the biological material as defined herein within a production protocol applied within the production line or in the stand-alone system. A unit for a pretreating may comprise cell wall digestion processes, supply of a buffer, agent etc. Preferably the at least one unit is a device for evacuolation of a plant cell line or a tank providing the evacuolated plant material.

In one embodiment, tank (B) and tank (A) are identical where it is desired to repeat more than one cycle of disruption. This embodiment applies for the stand-alone system as well as for the production-line. In the latter the production line comprises said circular disruption apparatus with the appropriate conduits and a mean to open the circular disruption apparatus to continue downstreaming process. After disruption is completed, the functional product as defined herein is moved to a separation device (C), preferably a centrifuge. In a preferred embodiment tank (B) is a device for separation, which preferably is a centrifuge. Said centrifuge is suitable to separate the at least one released, in particular intact, biologically active compartment enclosed by a lipid bilayer. Preferably the lipid bilayer of the separated compartment is intact. Thus, separation does not damage or disrupt the released biologically active compartments.

Another aspect of the present invention is a method for disruption of cell wall free cells, preferably of (mini)protoplasts and/or spheroplasts, comprising at least one biologically active compartment enclosed by a lipid bilayer, preferably said cells are in a range of equal to or more than 0.5 µm up to 200 µm, more preferably equal to or more than 5 µm up to 100 µm, 10 µm up to 100 µm, more preferably equal to or more than 10 µm up to 70 µm, more preferably equal to or more than 20 µm up to 70 µm, comprising the steps, preferably performed by use of any apparatus according to the present invention,
- Providing an aqueous medium comprising the cell wall free cells, preferably in a tank (A), preferably the aqueous medium comprises a biological material comprising the cell wall free cells and preferably has a viscosity of less than 20 mPa s (at 21°C) - less than 15 mPa s, less than 10 mPa s, in range of 0,5 up to 10 mPa s, preferably 0,5 up to 5mPa s -, preferably in tank (A) of the apparatus according to the invention,
- moving of the aqueous medium into the shear force generating device (S) as described herein, preferably at a predetermined speed as described herein for the (S) and/or (P)
- optionally regulation of the flow velocity and/or flow rate of the aqueous medium by means of a pump (P) as described herein, preferably by means of the pump speed as described herein,
- generating a region of a shear force within (S), preferably a region of shear forces and/or turbulence, more preferably in a centrifugal pump or in a rotor-stator system,
- exerting a shear force onto the aqueous medium comprising said cells and for a residence time that is sufficient to disrupt the cells without disrupting the at least one biologically active compartment, preferably exerting a shear force and/or centrifugal force within said region of shear force and/or turbulence,
- disrupting the cells without disrupting the lipid bilayer of the at least one biologically active compartment while the aqueous medium is passing (S),
- release of at least one intact biologically active compartment and its surrounding biologically active liquid, preferably into the aqueous medium,
- optionally collecting the at least one released intact biologically active compartment with its surrounding biologically active liquid, preferably in tank (B),
- separation of the at least one released intact biologically active compartment with its biologically active surrounding liquid, preferably by means of the separation device (C) according to the present invention, preferably wherein at least a fraction of debris ((mini)protoplast and/or spheroplast lipid bilayer) are removed,
- preferably obtaining of a fraction released intact biologically active compartments with its biologically active surrounding liquid, and
- optionally isolation of the at least one released intact biologically active compartment. The method according to the present invention is suitable to be performed by means of the stand-alone system or the production line as described alone. All embodiments of the apparatus apply accordingly to the method for disruption.

"Disrupting" the cells means that the lipid bilayer of the cell wall free cells, in particular protoplast and/or miniprotoplast or spheroplast, is "broken down" or "the cell is opened" in order to enable release of the biological content thereof as defined herein. "Disrupting" within the meaning of the present invention is a hydro-mechanical process by shear forces which is significantly different from a sonication that applies sound energy at difference frequencies (e.g. ultrasonic frequencies (> 20 kHz) in order to reduce particle size in a sample. It is also significantly different from the process of homogenization wherein two (non-soluble) liquids are mixed and treated in order to achieve a homogenous mixture, e.g. wherein uniformly dispersed particles are obtained. By means of homogenization also the biologically active compartments are disrupted.

Preferably said cell wall free cells are in the range of equal to or more than 5 µm up to 200 µm, more preferably equal to or more than 5 µm up to 100 µm, 10 µm up to 100 µm, more preferably equal to or more than 10 µm up to 70 µm, more preferably equal to or more than 20 µm up to 70 µm, preferably said cells comprise protoplast and/or miniprotoplast or spheroplast or other comparable biologically active structures of another species. Preferably, the aqueous medium comprising said cell wall free cells is essentially free of cellulose, essentially free of chitin, and/or essentially free of pectin. Preferably said cell wall free cells are derived from a plant cell, fungi yeast and/or an eukaryotic cells line, e.g. CHO. Said intact biologically active compartments and its surrounding biologically active liquid together form the functional product. The "biologically active liquid" comprises the cytosol of the cell wall free cell, preferably of (mini)protoplast and/or spheroplast, and the content of the aqueous medium. The cytosol comprises the cell line-specific, species-specific components e.g. enzymes, soluble proteins and/or small molecules. The aqueous medium may comprise nutrients and other process specific components. Separation of said functional product is performed by means of the separation device (C) according to the invention, preferably by centrifugation. Separation may be performed once or several times. Additionally, a step of isolation prior, after or instead of separation may be performed. Isolation is preferably performed after separation, more preferably by filtration and/or centrifugation of the separated functional product. The difference between separation and isolation is that the purpose of separation is to remove debris (cell membrane debris, and protoplast debris) from the functional product whereas the purpose of isolation is to isolate and potentially concentrate the desired fraction of biologically active compartments from the functional product. The desired "fraction" is defined by function and/or particles size of the biologically active compartments according to the invention. Preferably isolation is performed in order to obtain a "fraction" of biologically active microsomes and/or biologically active mitochondria.

A washing step may be further integrated into the method according to the present invention. Washing may be performed prior or after the at least one pretreating (e.g. evacuolation), prior or after the disruption according to the present invention, prior or after a further circle of disruption, prior or after isolation and/or prior or after the at least one separation. Thus, where appropriate a washing step may be integrated between any steps according to the present invention. Whether washing is necessary or desired depends on the volume achieved from the upstreaming step and on the volume necessary for the down streaming device/tank and/or step or for the final formulation. It may be desired to perform at least one washing step in order to isolate the at least one released intact biologically active compartment from the separated functional product. Washing is performed with an appropriate buffer/solution with lipid bilayer stabilizing properties in order to stabilize the isolated compartments, preferably microsomes and/or mitochondria. However, washing is not mandatory to obtain a "functional product" of good quality.

The functional product, any directly obtained process product or intermediate products obtained by the method according to the present invention as well as the separated functional product may be subjected to a further processing step. Thus, dependent on optionally isolation and/or washing and/or sterilization, a functional product of different degrees of purity is achieved (functional product with desired purity). After the desired degree of purity is achieved further processing may follow and comprise washing and/or mixing and/or supplementation and/or sterilization and/or freezing, drying, gas drying and/or lyophilization of the respectively achieved product. However, a subsequent step such as freezing, drying, gas drying and/or lyophilization may also be a step of the production line according to the invention. In this embodiment the final product is defined as the dried and/or the frozen lysate (final lysate) of the desired degree of purity.

"Isolation" means, that the product from the preceding steps, preferably the functional product is centrifugated and/or filtrated in order to achieve a higher content of the at least one biologically active compartment and preferably with a higher degree of purity compared to the purity of the intermediate product obtained from preceding step. By means of isolation the content of the surrounding biologically active liquid is reduced and the content of biologically active compartments increased. Preferably by means of isolation a desired "fraction" of biologically active compartments is isolated and/or concentrated, e.g. a fraction of those capable of ATP synthesis and/or energy regeneration or a fraction of those capable of at least one protein biosynthesis associated process comprising expression, transcription, translation, translocation, protein folding and/or protein modification. Preferably the ATP synthesis and/or energy regeneration active fraction comprises biologically active compartments in the range of 0.5-2 µm. The protein biosynthesis associated process active fraction comprises biologically active compartments in the range of 0.5-30 µm and are capable of at least one protein biosynthesis associated process comprising expression, transcription, translation, translocation, protein folding and/or protein modification. In the method according to the present invention isolation provides the desired fractions with the desired biological activity.

In another embodiment of the method according to the present invention a predetermined rotor tip speed of equal to or more than 2 m/s up to 50 m/s is applied by means of (S), preferably in the range of at least 4 m/sec and equal to or less than 32 m/sec. The appropriate range is adjusted to the scale of apparatus according to the present invention and used for the method according to the present invention. The rotor tip speed is preferably in the range of at least 2,5 m/sec and equal to or less than 38m/sec, 3 m/sec to 38m/sec, 3,5 m/sec to 38 m/sec, 3,5 m/sec to 36 m/sec, 3,5 m/sec to 34 m/sec, more preferably in the range of at least 4 m/sec and equal to or less than 32 m/sec. The aforementioned rotor tip speeds can be converted for analogues use of the alternative shear generating device (S) according to apparatus of the invention comprising a centrifugal pump, a high shear forces mixer (e.g. ultra turaxx) or a Rotor-Stator-mixer with a teeth-design or with a blade-design.

In another embodiment of the method according to the present invention, the shear force generating device S is a centrifugal pump, a high shear forces mixer or a Rotor-Stator-mixer with a teeth-design or with a blade-design, more preferably a Rotor-Stator-mixer with a teeth-design or with a blade-design (Fig. 2). Embodiments with different centrifugal pumps, rotor-stator mixers are described herein apply accordingly for the method.

Preferably, rotation of the rotor is adjusted, whereby generation of acceleration forces causing radial moving of the medium and axial suction of the medium is performed. Thereby the aqueous medium is forced towards the shearing gap and the cells are moving by means of shear and thrust forces between the rotor and the stator and affecting the cells at the shearing gaps. Thereby the cells are disrupted, in particular the lipid bilayer of said cells is disrupted without disruption of the lipid bilayer of the at least one comprising biologically active compartments.

In another embodiment of the method according to the present invention, the biologically active compartment, preferably the released biologically active compartment, is capable of ATP synthesis, energy regeneration, of at least one protein biosynthesis associated process, expression, transcription, translation, translocation, protein folding and/or protein modification. Preferably it is released into the aqueous medium ("directly obtained process product", "functional product"). Therefrom, different fraction can be separated and/or isolated. Preferably one fraction of isolated biologically active compartments according to the present invention is predominantly capable of ATP synthesis and/or energy regeneration and another fraction of isolated biologically active compartments according to the present invention is predominantly capable of at least one protein biosynthesis associated process, expression, transcription, translation, translocation, protein folding and/or protein modification. The later fraction is in particular capable of coupled transcription and co-translational translocation of the at least one complex proteins with the need of posttranslational modification PTM (e.g. GOx) and/or expressed transmembrane proteins (TP) (e.g. ACE2) into the at least one biologically active compartments, which preferably is a microsome, more preferably an endogenous microsome.

In another embodiment of the method according to the present invention, the at least one biologically active compartment has an average particle size in the range of at least 0,5 µm to less than 30 µm. More preferably the compartments are distinguishable in at least two fractions, comprising a fraction of predominantly biologically active compartments in the range of 0,5-2 µm and a fraction of predominantly biologically active compartments in the range of 0,5-30 µm. The fraction of predominantly biologically active compartments in the range of 0,5-2 µm is predominantly capable of ATP synthesis and/or energy regeneration and the fraction of predominantly biologically active compartments in the range of 0,5-30 µm is predominantly capable of at least one protein biosynthesis associated process, expression, transcription, translation, translocation, protein folding and/or protein modification, more preferably predominantly capable of coupled transcription and co-translational translocation of the at least one complex proteins with the need of posttranslational modification PTM (e.g. GOx) and/or expressed transmembrane protein into the at least one biologically active compartments, which preferably is a microsome, more preferably an endogenous microsome.

Preferably, a combination of two different biologically active compartments is obtained (fractions as described herein), wherein one fraction comprises biologically active compartments in the range of 0,5-2 µm and are at least capable of ATP synthesis and/or energy regeneration and the other fraction comprises biologically active compartments in the range of 0,5-30 µm and are capable of at least one protein biosynthesis associated process comprising expression, transcription, translation, translocation, protein folding and/or protein modification.

In another embodiment of the method according to present invention, the disruption efficiency is defined as the amount of disrupted cells in relation to the amount of not disrupted cells, preferably or alternatively as the amount of released biologically active compartments in relation to the amount of provided not disrupted cell wall free cells.

The respective amount is determined by counting of the provided cells wall free cells. The respective amount of biologically active compartments is counted, preferably by means of a microscopic analysis, an assay for a marker/component (e.g. a known and detectable transmembrane protein) of said biologically active compartments or by a particle flow measurement. The relation of an amount to another amount is represented as a percentage.

The method, wherein the disruption efficiency is the product of the shear gradient and the residence time of the aqueous medium in the shear force and/or turbulence region and defined as strong enough to disrupt or crack or break up the cell wall free cell ((mini)protoplast), preferably the (mini)protoplast's membrane, in order to release biologically active compartments, preferably of less than 70 µm in diameter. Preferably biologically active compartments are intact organelles comprising mitochondria, microsomes, nuclei, ER and/or Golgi from a plant (mini)protoplast. Preferably the isolated fraction comprises released intact microsomes and/or mitochondria from a plant (mini)protoplast but does not contain nuclear DNA.

Preferably said compartments are mitochondria and/or microsomes and most preferably the released biologically active compartment does not comprise nuclei and/or nuclear (karyologic) DNA, preferably the isolated fraction does not comprise cell membrane or fragments thereof, nuclei and/or nuclear DNA.

In another embodiment of the method according to the present invention, the method it is an integral method of a production process - as described in context of the apparatus according to the present invention, preferably of a cell lysate production process. The production process is a cell line production process and preferably with the purpose to produce cell lysate and/or to obtain a desired fraction from the cell line. Said desired fraction is a certain soluble protein, a transmembrane protein embedded in the lipid bilayer of the biologically active compartment according to the present invention and/or another component, main or side product of the production process. More preferably the production process is a plant cell lysate production process, most preferably of Nicotiana tabacum BY2, for the manufacture of a lysate for use for cell-free protein synthesis.

Another aspect of the present invention is a composition comprising at least one biologically active compartment and its surrounding biologically active liquid, wherein the liquid and/or the compartment enables at least one step of a protein biosynthesis, preferably obtained by the method according to the present invention. More preferably obtained by the method according to the present invention which is performed by use of the apparatus according to the present invention, stand-alone or as part of an inline production. All embodiments of the at least one biologically active compartment, fractions and/or its surrounding biologically active liquid apply accordingly for the composition according to the present invention.

Another aspect of the present invention is an isolated biologically active compartment enclosed by a lipid bilayer and exhibiting at least a capacity in ATP synthesis and/or energy regeneration and/or at least a capacity in at least one protein synthesis process preferably comprising transcription, translation, post-translational modifications, protein folding and/or translocation. Preferably said isolated biologically active compartment enclosed by a lipid bilayer is obtained by the method according to the present invention and/ preferably by use of the apparatus according to the present invention. All embodiments of the at least one biologically active compartment, fractions and/or its surrounding biologically active liquid apply accordingly for the isolated biologically active compartment enclosed by a lipid bilayer according to the present invention.

In another embodiment of the present invention, the isolated biologically active compartment enclosed by a lipid bilayer or the composition comprising at least one biologically active compartment, respectively comprises at least one transmembrane protein, at least one inner and/or outer membrane-associated proteins, and/or at least one soluble protein within the inner space.

Preferably said biologically active compartment is obtained by the method according to the invention and preferably by use of the apparatus according to the present invention.

### Description of the drawings

**Fig. 1****:** shows a schematic arrangement of the apparatus according to the present invention, wherein (S) is the shear generating device, (P) the pump, tank A for the provision of an aqueous medium, a tank B for collecting the downstreaming process product and (C) is the separation device, preferably a centrifuge.
**Fig. 2****:** shows schematically the A) blade-design and B) teeth-designs of a Rotor-Stator-mixer of a shear generating device (S) according to the invention.
**Fig. 3****:** Work flow (protocol) of the method according to invention with embodiments, wherein it is integrated into production line as described herein. The different starting materials ("Starting material comprising a cell line of interest"), an optional "pretreating" as well as the starting material for the method of the present invention ("Starting material comprising biological material") is shown. The herein defined products are shown in correlation to the respect step described herein. The optional steps of washing and/or sterilization ("washing/sterilization") may be integrated between any step. Sterilization may be applied to components, devices and/or units supplemented or mounted to the apparatus according to the invention.

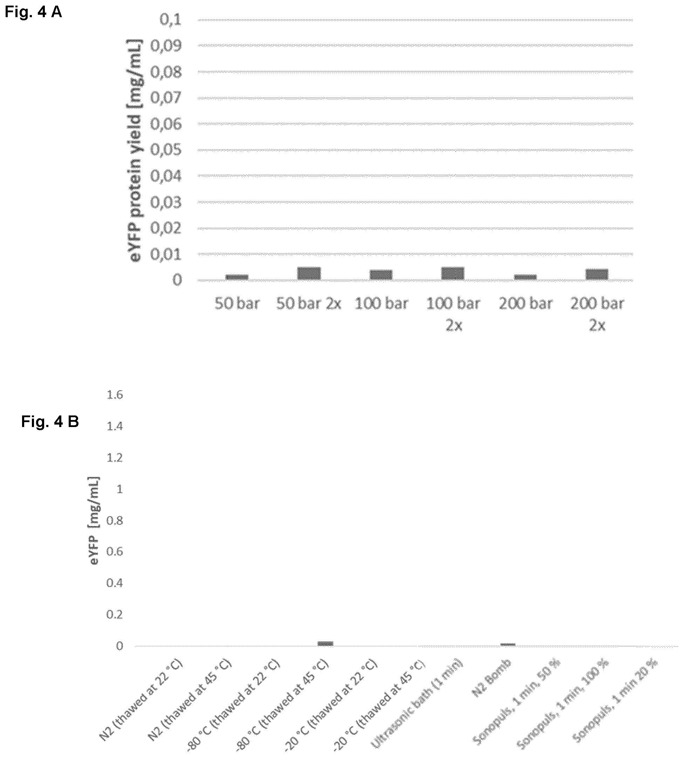

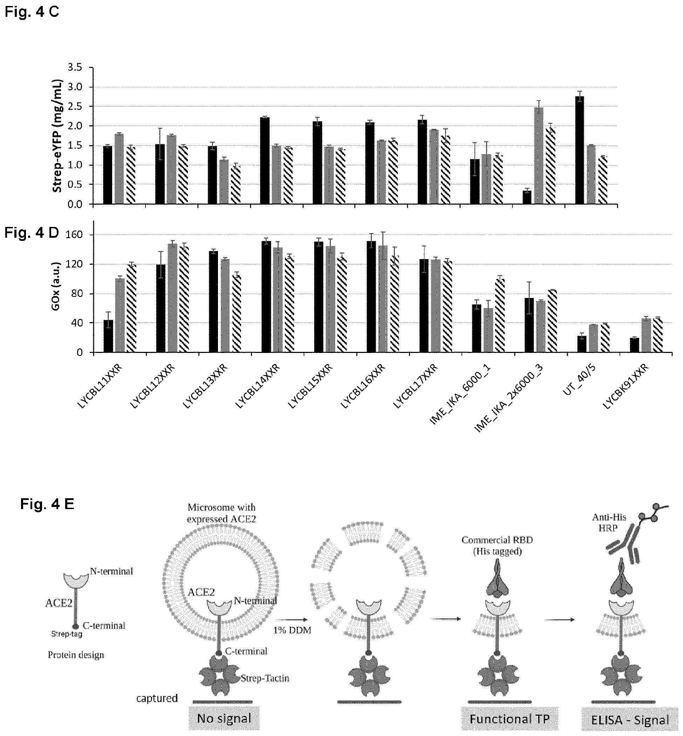

**Fig. 4****:** eYFP yield produced by a cell lysate comprising biological active compartments such as mitochondria and microsomes achieved from different disruption methods. The method of protein synthesis method is feasible in order to prove efficient cell disruption while ensuring a biological active lysate. Disruption efficiency as function of protein production. A: Disruption with a Highpressure homogenizer (Microfluidizer: Model M-110L; LM-10) B: Disruption efficiency of N2 bomb (N2), Freeze Thaw (-80/-20°C), Utrasonication (Ultrasonic), Sonication (Sonoplus) C: Disruption efficiency of IKA magic lab under different conditions (table 2) as function eYFP production (10 ng/µL (black bar), 20 ng/µL (grey bar), 40 ng/µL (dashed bar)). D: Disruption efficiency of IKA magic lab under different conditions as function of GOx production (5 ng/µL (black bar), 10 ng/µL (grey bar), 20 ng/µL (dashed bar)). E: Assay for disruption efficiency of the method according to the present invention as function of production of an functional transmembrane protein (TP), here angiotensin converting enzyme ACE2, by binding to its ligand (commercial Receptor binding domain RBD).

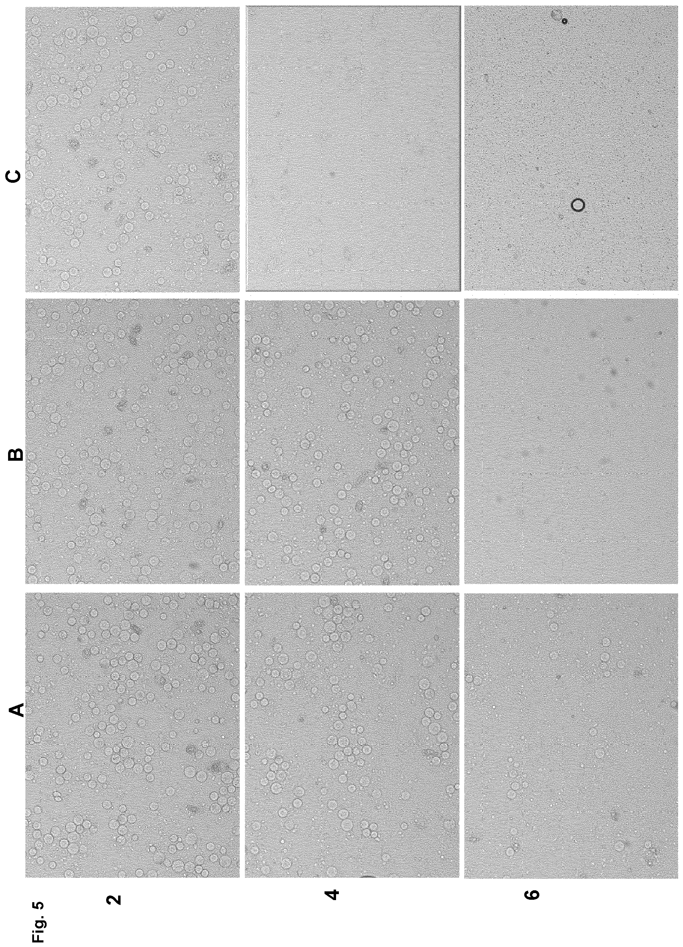

**Fig. 5****:** Microscopic evaluation (40x) of lysates of BY-2 cell line ; **A:** pumping speed: 40 (30 mL/min) with ultra Turrax speed 2, 4 or 6, **B:** pumping speed: 30 (22.5 mL/min) with ultra Turrax speed 2, 4 or 6, C: pumping speed: 20 (15 mL/min) with ultra Turrax speed 2, 4 or 6; For A, B and C with a Ultra turrax speed of 2 three distinct fractions are visible, For A, B and C with a Ultra turrax speed of 4 three fractions are visible but less distinct than with a Ultra turrax speed of 2; For A, B and C with a Ultra turrax speed of 4 only two distinct fractions are visible.

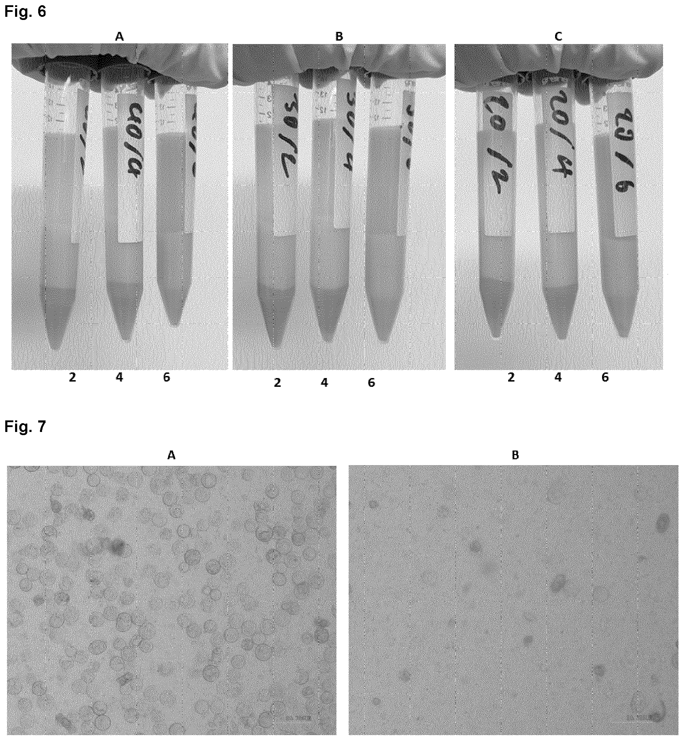

**Fig. 6****:** Macroscopic evaluation of lysates derived from a BY-2 cell line after disruption and centrifugation at 1,500xg for 15 min; **A:** pumping speed: 40 (30 mL/min) with ultra Turrax speed 2, 4 or 6, **B:** pumping speed: 30 (22.5 mL/min) with ultra Turrax speed 2, 4 or 6, C: pumping speed: 20 (15 mL/min) with ultra Turrax speed 2, 4 or 6; For A, B and C with a Ultra turrax speed of 2 three distinct fractions are visible. The fraction on the bottom comprises the non-disrupted cell wall free cells (mini)protoplasts, the fraction above comprises the non-desired fraction to be separated comprising nuclei and debris (lipid bilayer debris, and (mini)protoplast debris), the upper fraction comprises the desired fraction (lysate) comprising biologically active compartments and surrounding biologically active liquid (ribosomes, mitochondria, microsomes. etc.). For A, B and C with a Ultra turrax speed of 4, three fractions are visible but less distinct than with a Ultra turrax speed of 2; For A, B and C with a Ultra turrax speed of 4 only two distinct fractions are visible.
**Fig. 7****:** Microscopic evaluation after disruption with IKA magic LAB with a single rotor/stator at a rotation speed of 10000rpm and a feed speed of 37 mL/min after one pass.

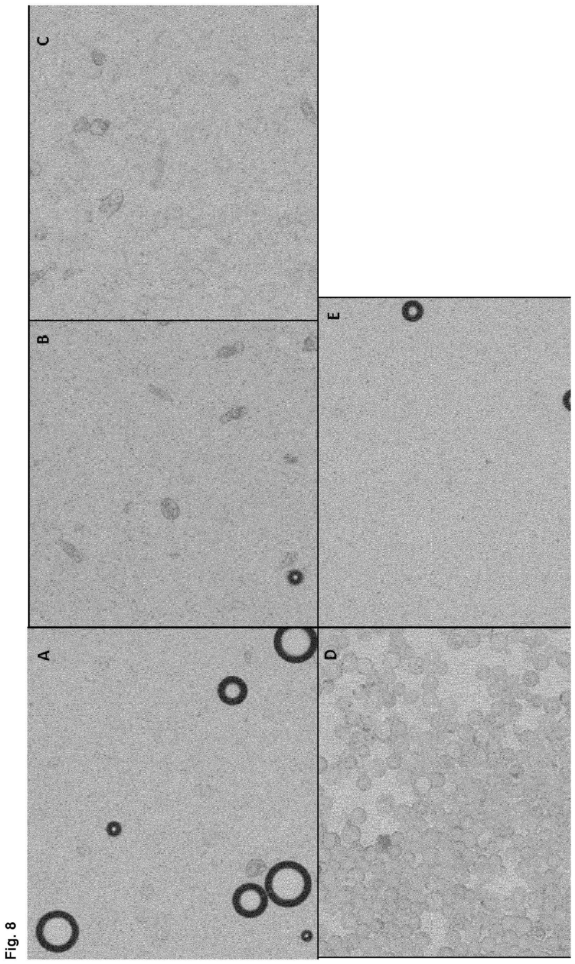

**Fig. 8****:** Microscopic evaluation after disruption with IKA magic LAB with a triple or single rotor/stator with a feed speed of 40 mL/min respectively. A) triple rotor/stator, 6000rpm after two passes, of B) single rotor/stator, 6000rpm after two passes, of C) triple rotor/stator, 6000rpm after one pass, D) Feed E) triple rotor/stator, 9000rpm after one pass.

### References

**Buntru** et al. (2014) - M. Buntru, S. Vogel, H. Spiegel and S. Schillberg; Tobacco BY-2 cell-free lysate: an alternative and highly-productive plant-based in vitro translation system. BMC Biotechnology 2014, 14:37. https://doi.org/10.1186/1472-6750-14-37
**Buntru** et al. (2015) - M. Buntru, S. Vogel, K. Stoff, H. Spiegel, S. Schillberg; A Versatile Coupled Cell-Free Transcription-Translation System Based on Tobacco BY-2 Cell Lysates. Biotechnology and Bioengineering, Vol. 112, No. 5, May, 2015. DOI 10.1002/bit.25502
**Espinoza** et al. - C.J.U. Espinoza, F. Alberini, O. Mihailova, A.J. Kowalski, M.J.H. Simmons; Flow, turbulence and potential droplet break up mechanisms in an in-line Silverson 150/250 high shear mixer. Chemical Engineering Science: X 6 (2020) 100055. https://doi.org/10.1016/j.cesx.2020.100055
**Håkansson** - Andreas Håkansson; Rotor-Stator Mixers: From Batch to Continuous Mode of Operation-A Review. Processes 2018, 6, 32; doi:10.3390/pr6040032
**Zhang** et al. - Jinli Zhang*, Shuangqing Xu, Wei Li; High shear mixers: A review of typical applications and studies on power draw, flow pattern, energy dissipation and transfer properties. Chemical Engineering and Processing 57- 58 (2012) 25- 41 http://dx.doi.org/10.1016/j.cep.2012.04.004

### Examples

### Example 1 - proof of concept

### 1.1 Experimental setup

A peristaltic pump, here watson marlow 120u/dv pump with an inner tubing diameter of 3.175 mm, was used and as a shear generating device a Ultra turrax, (IKA ULTRA-TURRAX^{®} T 10), was combined. Three different pumping speeds and three different ultra turrax speeds were applied (each around 10 mL). The starting material was a pre-treated, aqueous medium comprising evacuolated, cell wall free BY-2 cells derived from a suspension cell culture of N. tabacum (Buntru et al. 2014, 2015) supplemented with 1.5-times (v/v) TR buffer. The starting material was kept cool (below 10°C) during the experiment.

For the aqueous medium a TR buffer that contained 30 mM HEPES KOH buffer pH 7.6, 40 mM Potassium glutamate, 0,5 mM magnesium glutamate, and 2 mM DTT) was used. After the completed method, the directly obtained process product (as defined herein) comprises the TR-buffer, the released biologically active compartment and/or its surrounding.

**Table 1: Applied shear forces and flow rates**

| | **Sample name** | **Pump speed** | Flow rate | **Ultra turrax speed** |
|---|---|---|---|---|
| Fig.5 | | **rpm** | **mL/min** | **Arbitrary** |
| **Colum \ Row** | | | | **2/4/6** |
| **A\2** | 40/2 | 40 | 30 | 2 |
| **A\4** | 40/4 | 40 | 30 | 4 |
| **A\6** | 40/6 | 40 | 30 | 6 |
| **B\2** | 30/2 | 30 | 22.5 | 2 |
| **B\4** | 30/4 | 30 | 22.5 | 4 |
| **B\6** | 30/6 | 30 | 22.5 | 6 |
| **C\2** | 20/2 | 20 | 15 | 2 |
| **C\4** | 20/4 | 20 | 15 | 4 |
| **C\6** | 20/6 | 20 | 15 | 6 |

### 1.2 Results

The microscopic evaluation (Fig. 5) shows the directly obtained process product after disruption with an ultra turrax and pump as presented in table 1. Fig.5 shows that both pump speed and/or flow rate and Ultra turrax speed have an effect on disruption, with a higher ultra turrax speed (top to bottom in figure panels) and a lower pump speed and/or flow rate (left to right in figure panels) both individually leading to a more disrupted process product (lower content of visible microstructures). The most extreme setting of both parameters is shown in Fig. 5 C/6, where essentially all (mini)protoplasts of BY-2 were disrupted. The macroscopic evaluation of lysates of BY-2 cell line after disruption and centrifugation at 1,500xg for 15 min (Fig. 6) shows that at a pumping speed of 30, 22.5 or 15 mL/min and with a ultra Turrax speed of 6, respectively, all (mini)protoplasts) were successfully disrupted. Only two fractions, one comprising the biological active compartments (mitochondria and microsomes) and surrounding biologically active liquid, and one comprising the nuclei and/or debris of the (mini)protoplasts are visible. A pumping speed of 30, 22.5 and 15 mL/min with a ultra Turrax speed of 2 and 4 was not sufficient to disrupt the whole biological material.

### Example 2 - IKA magic LAB trial

### 2.1 Experimental setup

### Apparatus for disruption

A peristaltic pump, here watson marlow 120u/dv pump, was used and as a shear generating device the IKA magic LAB was combined. If not stated otherwise, the same set-up was used (Fig. 5, 6, 7 and 8).

| IKA module | Single rotor/stator or triple rotor/stator |
|---|---|
| Feed speed | 37 mL/min |
| # passes | 1 |
| rotor speed | 10,000 rpm |

For the later biosynthesis assays (2.2.1 and 2.2.2) the lysate was produced by performing the disruption by means of IKA magic lab with the experimental setup as follows.

**Table 2: Applied shear forces and flow rates**

| Lysate LOT | pump flow (mL/min) | pass | IKA magic LAB rotor speed (rpm) |
|---|---|---|---|
| **LYCBL11XXR** | 40 | 1 | 10000 |
| **LYCBL12XXR** | 40 | 1 | 6000 |
| **LYCBL13XXR** | 40 | 1 | 3000 |
| **LYCBL14XXR** | 40 | 1 | 3000 |
| **LYCBL15XXR** | 40 | 2 | 3000 |
| **LYCBL16XXR** | 40 | 3 | 3000 |
| **LYCBL17XXR** | 40 | 4 | 6000 |
| **IME_IKA_6000_1** | 40 | 1 | 6000 |
| **IME_IKA 2x6000_3** | 40 | 2 | 6000 |
| **UT 40/5** | Ultra turrax - down-scaled IKA | | |
| **LYCBK91XXR** | Dounce homogenizer | | |

The same starting material was used as described above and was kept cool (below 10°C) during the experiment.

### 2.2 Proof of disruption efficacy:

Disruption efficacy is evaluated visually (Fig. 6), by microscope (Fig. 5, 7 and 8) and by means of protein synthesis.

### 2.2.1 Assay to proof expression of a cytosolic protein, eYFP as described in Buntru et al. (page 9)

As marker protein the enhanced yellow fluorescent protein (eYFP) is used. It is expressed in the lysate outside of the microsome and does not need posttranslational modification (PTM) mediated by microsome. However, an active protein synthesis and energy regeneration machinery (ribosomes, mitochondria, etc.) is necessary. The higher the protein expression of eYFP (yield), the higher the measurable fluorescence. Alternative model proteins which are known in this context area firefly luciferase (FFLuc) and Renilla reniformis luciferase (Buntru et al 2014).

### 2.2.2 Assay to proof microsomal expression with posttranslational modification and folding (GOx)

In order to proof that the released microsomes are active and capable of posttranslational modification and folding the expression of the multi-domain glycoprotein (a homodimer comprised of 80kDa monomers that are covalently linked by disulfide bond and each possessed of 8 N-glycosylation sites) glucose oxidase (GOx) from Aspergillus niger is tested. For expression, the template encoding for the protein GOx was cloned into plasmid pALiCE02. After the post-incubation lysate samples were treated with 0.5 % DDM from a 5 % DDM stock in PBS for 10 minutes at room temperature. GOx standard from Aspergillus niger (Sigma Aldrich) was used to prepare a calibration curve in a range from 0-500 µg/ml. Samples and standard were diluted 1:2500 in assay buffer consisting of 0.33 M glucose, 0.67 mM ABTS and 1.67 U/ml HRP (Sigma Aldrich) in 0.1 M potassium phosphate buffer at pH 6 in transparent 96 well plates. Absorbance over time at 420 nm was measured in an infinite PRO Tecan plate reader for 15 minutes. The linear absorbance increase over time of the calibration samples was used to calculate sample GOx activity.

### 2.2.3 Assay to proof expression of transmembrane proteins embedded in the lipid bilayer of the microsome (Fig. 4 E).

Another method in order to prove intact microsomes comprising correctly folded and in the lipid bilayer embedded transmembrane proteins is the expression of a model transmembrane protein, e. g. Angiotensin converting enzyme ACE2, by use of the respective lysate after disruption. Since the so called Covid pandemic ACE2 is known being expressed on alveolar epithelial cells and capillary endothelial cells and actin as the cellular doorway of SARS-CoV-2 allowing the infection of a cell. Commercially available ligands (RBD) are well known. For the assay the ACE2 encoding template was cloned into the vector pALiCE02 (LenioBio GmbH) and a Strep-Tag^{®} II was fused C-terminally to the DNA templates for protein orientation and later capturing to the microtiter plate. Melittin signal peptide sequence (MSP) was N-terminally fused for microsomal targeting. ACE2 was expressed and translocated into the intact microsome, wherein the binding site at the N-terminus of the ACE2 is inside of the microsome and the C-terminus remained outside enabling capturing. 1% DDM in PBS was added to each well, and the plate was incubated for 15 minutes at room temperature. The microtiter plate was washed (3 x with PBS - 0.05% Tween (v/v) (PBST) and blocked adding 1x Blocking buffer (ab126587, Abcam) to prevent unspecific binding (1h, RT) and again washed. For capturing of the ACE2 embedded in the microsome and protein binding analysis, commercially available RBD SARS-CoV Spike/RBD Protein (RBD, His Tag) (40150-V08B2, SinoBiological) were diluted to 2.5 µg/ml in Blocking buffer. Plates were incubated at room temperature for 1 hour and followed by a washing step. In order to enable binding to ACE2 the microsomes were treated with 1% DDM to disrupt the captured microsomes. For protein detection, a detection antibody (HRP labelled) that binds to the protein was added. For ACE2-commercial RBD binding detection His Tag Horseradish Peroxidase-conjugated antibody (MAB050H, Bio-Techne) 1:4000 and StrepMAB-Classic - HRP (2-1509-001, IBA-lifesciences) 1:15.000 in Blocking buffer were added. Plates were incubated (1h, RT), then washed and indirect ELISA (by measuring sample absorbance) follows to determine the protein's structure and binding efficiency. Detection of absorbance (e.g. (650nm) TECAN Infinite M1000 Pro machine, Tecan i-Control 2.0 software) proves expression of correctly folded ACE2 because of effective binding to its ligand RBD of SARS-CoV-1. High absorbance values prove high yields of ACE2 expression, posttranslational modification, folding and embedment into the lipid bilayer of the microsome as precondition for RBD ligand binding. Therefore, this assay is suitable to proof the release of biologically active compartments according to the invention as shown in Fig. 4 E.

### 2.3 Results

### 2.3.1 Homogenization, Sonication etc. vs. shear force disruption

The disruption method by means of the Microfluidizer: Model M-110L; LM-10 was performed in order to evaluate whether homogenization, as used in the prior art in other technical fields, could enable lysate production with high yields of biologically active components. However, only low yields of expressed protein of below 0.01 mg eYFP/ml were observed (Fig. 4 A). Consequently, only a low content of biologically active components was achieved in the lysate produced by homogenization. Other prior art methods such as sonication etc were tested accordingly. It is shown (Fig. 4 B) that the yield of expressed eYFP is very low which is due to the low content of biologically active components of the lysate. The same assay was/will be applied on the products achieved by the methods performed by use of the IKA magic LAB, IKA UTL 25, Levitronix^{®} puraLev i100 SU and Ultra turrax, (IKA ULTRA-TURRAX^{®} T 10). Fig. 4 C shows the eYFP yield produced by a lysate after different disruption methods.

The disruption method according to the present invention by means of a shear generating device (S), here IKA magic lab, is shown in Fig. 4C and Fig. 4D. Compared to the results shown in Fig. 4 A and B, the achieved protein yield was between 1 mg/mL and 2.8 mg/mL eYFP depending on the disruption conditions and the used plasmid concentration (10, 20, 40 ng/µL from left to right column Fig. 4 C. The data shows that various disruption settings of IKA magic lab lead to biologically active compartments capable of reaching high target protein titers in lysate reactions. Further the Assay with GOx shows that the obtained lysate depending on the disruption conditions and the used plasmid concentration (5, 10, 20 ng/µL from left to right column, Fig. 4 D) also enabled production of the complex GOx protein with posttranslational modification mediated by intact microsomes between 40 and 150 a.u. (arbitrary units) GOx (Fig. D).

### 2.3.2 Microscopic evaluation (Fig. 7)

Fig. 7 represents microscopic evaluation (x100) of the starting material before disruption (Fig. 7A) and after disruption with one pass and 6,000 rpm (Fig. 7 B). The picture shows that the majority of cell wall free cells have been disruption with these settings. As not all cell wall free cells were disrupted with these settings, it also shows that the exerted forces were low enough to not destroy the cells compartments but release them biologically active.

### 2.3.3 Results (Fig. 8)

| **Pass #/ other** | **Feed speed** | **rotor speed** | **IKA module** | **Fig. 8** |
|---|---|---|---|---|
| **2** | 60 mL/min | 6,000 rpm | Triple rotor/stator | A |
| **2** | 60 mL/min | 6,000 rpm | Single rotor/stator | B |
| **1** | 60 mL/min | 3,000 rpm | Triple rotor/stator | C |
| **1 / Feed tube and IKA filled with TR buffer** | 60 mL/min | 9,000 rpm | Triple rotor/stator | E |
| **0/ Feed** | n.a. | 0 rpm | n.a. | D |

Fig. 8 represents microscopic evaluation of the starting material and the impact of the disruption settings on the amount of disrupted cell wall free cells. Fig. 8 D shows the cell wall free cells before disruption. Fig. 8 C shows that the majority of cell wall free cells are still intact after a single pass with 3,000 rpm rotor speed. In contrast, Fig. 8 A, B, E shows that the cell wall free cells were disrupted. The comparison of Fig. 8 A and 8 B shows the impact of the rotor stator configuration. It shows that with the same settings other than a triple vs. a single rotor/stator module, different shear forces can be achieved to finely tune the appropriate force to disrupt cell wall free cells.

### 3. Summary

Presently, it was shown by the expression of eYFP and GOx that the functional lysate is obtained by the disruption method according to the present invention. Not only the protein biosynthesis machinery is complete and capable to express a soluble protein (eYEP) but also the posttranslational modification of the multidomain protein GOx takes place. Consequently, the disruption method and the apparatus according to the present invention allows the production of functional lysate at various scales for use of biosynthesis of recombinant proteins. Presently, a successful scaling of a crucial step of the lysate production, improvement of the production efficiency, while ensuring and improving the quality of the directly obtained process product has been successfully shown.

### Example 3 -Blade and teeth design trial

In-line Production by use of IKA UTL 25 Inline to show the impact of the blade design compared to the teeth design of the rotor (Fig. 2)

### Experimental set up

Peristaltic pump, e.g. watson marlow 120u/dv pump in combination with the shear generating device with a) blade design and with b) the teeth design respectively. The same starting material as described in Example 1 and 2. Adjustment of the rotor speed between 3,000 - 15,000 rpm, e. g. 3000, 4000, 5000, 6000, 7000, 8000, 9000, 10000, 11000, 12000, 13000, 14000 and 15000 respectively. Adjustment of the pump in a range between 10 - 100 mL/min, respectively:

| | **flow rates** ml/min | | | | | | | | |
|---|---|---|---|---|---|---|---|---|---|
| | 10 | 8-14 | 24-29 | 29-47 | 47-58 | 58-67 | 67-85 | 85-94 | 94-100 |

### Example 4 - Centrifugal pump trial

### Experimental set up

A centrifugal pump, e.g. the Levitronix^{®} puraLev i100 SU instead of a rotor-stator mixer. The same starting material as described in Example 1 and 2. The centrifugal speed (in rpm) will be higher and the feed rate of the peristaltic pump will be lower, as the exerted forces on the cell wall free cells will be lower with a centrifugal pump compared to a rotor-stator mixer. 4,500 - 9,000 rpm centrifugal speed will be used with peristaltic pump speeds of 1 - 50 mL/min. Furthermore, multiple passes through the centrifugal pump will be used.

### Example 5 - Scale-up trial

To implement the residence time of the cell wall free cells in the shear force field as scale-up parameter, the Ultra turrax, (IKA ULTRA-TURRAX^{®} T 10), the IKA UTL 25 inline and, the IKA magic LAB will be used in parallel experiments to confirm the residence time as a scale-up criterion together with shear force in the shear force field.

### Example 6 - Viscosity determination

Viscosity determination and adjustment of different starting materials that can be disrupted with the described method and by the use of one embodiment of the apparatus according to the present invention. Feasible viscosities with the shear generating devices will be tested. A Viscosity of appr. 0,5 cP up to 5 cP is an aqueous medium and feasible with each embodiment of the inventive apparatus and method. However, viscosities of more than 5 up less than 15 mPa s can represent higher concentrated cell culture media comprising the biological material to be disrupted, e.g. after centrifugation.

### Example 7 - Isolation experiment, followed by determination of amount of compartment, amount per cell wall free cell and qualification.

### Fractionation of biological active compartments by centrifugation.

Done by assay for marker of the component and determination of nuclear DNA amount depending on disruption settings

### Example 8 - Disruption of yeast protoplasts, CHO and other species

A comparison of the most common cell lines (e.g. as described in Buntru et al 2015 and Buntru et al 2014, page 2) used in the field of cell free protein synthesis are disrupted as described herein in order to release and separate biologically active compartments and a biological active liquid suitable for cell free biosynthesis of a recombinant protein.

## Claims

1. An apparatus for disruption of cell wall free cells without disrupting comprising biologically active compartments enclosed by a lipid bilayer, the apparatus comprises
- a shear force generating device (S) for generating a region of a shear force suitable for disrupting the cells,
- a tank A for the provision of the cells,
- optionally a pump (P) positioned upstream of (S) or downstream of (S) for pumping an aqueous medium comprising said cells with an adjustable flow rate and/or flow velocity,
- at least one conduit connecting the tank A and the shear force generating device (S) and/or at least one conduit connecting (S) with a tank B,
- a tank B for collecting a product from the previous steps comprising at least one released biologically active compartment, and
- a separation device (C) to separate the disrupted cells and fragments thereof from the at least one released biologically active compartment and/or its surrounding biologically active liquid phase.

2. The apparatus according to claim 1, wherein the shear force generating device (S) is suitable and controllable to generate a shear forces sufficient to disrupt the cells without damaging the lipid bilayer of the at least one comprising biologically active compartment.

3. The apparatus according to claim 1 or 2, wherein the pump is suitable to adjust a residence time of the aqueous medium comprising the cells within (S) independently from the shear force adjusted and generated by (S), wherein the residence time is the duration for which the shear force is exerted onto the aqueous medium comprising the cells.

4. The apparatus according to any one of the claims 1 to 3, wherein the apparatus is an integral apparatus of a production line, preferably of a closed and sterile production line.

5. A production line comprising the apparatus according to any one of the claims 1 to 4, wherein tank A is placed downstream of at least one unit for a pretreating of a biological material and the conduit connects tank A with S and wherein tank B is placed downstream of S and optionally a pump P is placed downstream or upstream of S.

6. A method for disruption of cell wall free cells comprising at least one biologically active compartment enclosed by a lipid bilayer comprising the steps
- Providing an aqueous medium comprising the cell wall free cells, preferably in a tank A,
- moving of the aqueous medium into the shear force generating device (S) according to claim 1 or 2,
- optionally regulation of the flow velocity and/or flow rate of the aqueous medium by means of a pump (P) according to claim 3,
- generating a region of a shear force within (S),
- exerting a shear force onto the aqueous medium comprising said cells and for a residence time that is sufficient to disrupt the cells without disrupting the at least one biologically active compartment,
- disrupting the cells without disrupting the lipid bilayer of the at least one biologically active compartment while the aqueous medium is passing (S),
- release of at least one intact biologically active compartment and its surrounding biologically active liquid,
- optionally collecting the at least one released intact biologically active compartment with its surrounding biologically active liquid, preferably in tank B,
- separation of the at least one released intact biologically active compartment with its biologically active surrounding liquid, and
- optionally isolation of the at least one released intact biologically active compartment.

7. The method according to claim 6, wherein a predetermined rotor tip speed of equal to or more than 2 m/s up to 50 m/s is applied by means of (S).

8. The method according to any one of the claims 6 to 7, wherein the shear force generating device (S) is a centrifugal pump, a high shear forces mixer or a Rotor-Stator-mixer with a teeth-design or with a blade-design.

9. The method according to any one of the claims 6 to 8, wherein the biologically active compartment is capable of ATP synthesis, energy regeneration, of at least one protein biosynthesis associated process, expression, transcription, translation, translocation, protein folding and/or protein modification.

10. The method according to any one of the claims 6 to 9, wherein the at least one biologically active compartment has an average particle size in the range of at least 0,5 µm to less than 30 µm.

11. The method according to any one of the claims 6 to 10, wherein the disruption efficiency is defined as the amount of disrupted cells in relation to the amount of not disrupted cells, preferably or alternatively as the amount of released biologically active compartments in relation to the amount of provided not disrupted cell wall free cells.

12. The method according to any one of the claims 6 to 11, wherein it is an integral method of a production process, preferably of a cell lysate production process.

13. A composition comprising at least one biologically active compartment and its surrounding biologically active liquid, wherein the liquid and/or the compartment enables at least one step of a protein biosynthesis.

14. An isolated biologically active compartment enclosed by a lipid bilayer and exhibiting at least a capacity in ATP synthesis and/or energy regeneration and/or at least a capacity in at least one protein synthesis process preferably comprising transcription, translation, post-translational modifications, protein folding and/or translocation.

15. The biologically active compartment of claim 13 or 14, wherein it comprises at least one transmembrane protein, at least one inner and/or outer membrane-associated proteins, and/or at least one soluble protein within the inner space.
